# EUROPÄISCHE PATENTSCHRIFT

(11) **EP 2 055 307 B1**
(45) Veröffentlichungstag und Bekanntmachung des Hinweises auf die Patenterteilung: **14.12.2011**
(21) Anmeldenummer: 08017619.1
(22) Anmeldetag: 08.10.2008
(51) Int. Cl.: A61K 8/97, A61K 36/82, A61K 36/57

(54) **Wirkstoffkombinationen aus Anisfruchtextrakt und Weissem Teeextrakt**
Active agent combinations made from anise fruit extract and white tea extract
Combinaisons de matières actives constituées d'un extrait de fruit anisé et d'un extrait de thé blanc

(30) Priorität: 01.11.2007 DE 102007052536
(43) Veröffentlichungstag der Anmeldung: 06.05.2009
(73) Patentinhaber: Beiersdorf AG, 20245 Hamburg (DE)
(72) Erfinder: Fänger, Sabine, 22763 Hamburg (DE); Kröpke, Rainer, 22869 Schenefeld (DE); Mummert, Christopher Dr., 29553 Bienenbüttel (DE); Schulz, Jens Dr., 25462 Rellingen (DE)
(74) Vertreter: Wilke, Jochen

(56) Entgegenhaltungen:
- WO-A-2004/012650
- WO-A-2006/000226
- WO-A-2006/128779
- WO-A-2006/128780
- WO-A-2007/051297
- US-A1- 2004 175 351
- US-A1- 2005 084 546

## Beschreibung

Die vorliegende Erfindung betrifft kosmetische Zubereitungen enthaltend Wirkstoffkombinationen aus einer wirksamen Menge an Anisfruchtextrakt und Weißem Teeextrakt.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können;
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewusstsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten. Dabei sollten die Zubereitungen insbesondere auch die Hautfeuchtigkeit verbessern.

Es hat sich überraschenderweise herausgestellt, dass eine kosmetische Zubereitung, dadurch gekennzeichnet, dass sie eine kosmetisch wirksame Menge an Anisfruchtextrakt und Weißen Teeextrakt enthält, den Nachteilen des Standes der Technik abhilft.

Die Verwendung erfindungsgemäßer Zubereitungen mit einem kosmetisch wirksamen Gehalt an Anisfruchtextrakt und Weißen Teeextrakt - z. B. in Form der genannten Beispiele - führt in überraschender Weise zu einer erheblichen Verbesserung des Aussehens der Haut, insbesondere werden durch Anwenden erfindungsgemäßer Zubereitungen
- die Barriereeigenschaften der Haut erhalten oder wiederhergestellt,
- besser der Hautaustrocknung entgegengewirkt sowie
- die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an Anisfruchtextrakt und Weißen Teeextrakt enthalten, zur Steigerung der Hautfeuchtigkeit bzw. zur Befeuchtung der Haut.

Die erfindungsgemäßen Zubereitungen eignen sich darüber hinaus hervorragend zur Pflege sensibler Haut.

Es war ferner überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Bevorzugt enthalten kosmetische oder dermatologische Zubereitungen gemäß der Erfindung 0,001 bis 30 Gew.-%, bevorzugt 0,1 bis 15 Gew.-%. besonders bevorzugt 0,5 bis 5 Gew.-% an Anisfruchtextrakt und Weißen Teeextrakt, jeweils bezogen auf das Gesamtgewicht der Zubereitung.

Vorteilhaft werden Gewichtsverhältnisse von Anisfruchtextrakt und Weißen Teeextrakt aus den Bereichen 1 : 10 bis 10 : 1, bevorzugt von 1 :5 bis 5 :1,besonders bevorzugt von 1 : 2 bis 2 : 1, gewählt.

Es ist erfindungsgemäß insbesondere vorteilhaft, einen wäßrigen Frucht-Extrakt aus Anis (INCI: Pimpinella anis fruit extract), der reich an anorganischen Mineralien wie Natrium-und Magnesium-lonen, besonders an Kalium-lonen ist und mit Butylenglycol 0,36% und Parabenen 0,14% konserviert ist und durch enzymatische Hydrolyse in Wasser solubilisierter Anis-Früchte erhältlich ist, wobei das Verhältnis von Rohmaterial zu Extrakt etwa 1 : 2 beträgt und unter dem Handelsnarnen Bioxilift ® bei der Gesellschaft SILAB, (Brive Cedex, France) erhältlich ist.

Bei Anisfruchtextrakt (Bioxylift ®, Fa. SILAB) handelt es sich um einen wäßrigen Frucht-Extrakt aus Anis Der Extrakt weist eine bernsteinartige Farbe auf und hat einen charakteristischen Anis-Geruch. Er ist reich an anorganischen Mineralien wie Natrium-und Magnesiumionen, besonders aber an Kaliumionen.

Die spezifische Zusammensetzung beträgt:

| | |
|---|---|
| Trockenmasse: | 40 - 60 g/l |
| Mineralasche | 11 - 18 g/l |
| Gesamtprotein | 12 - 20 g/l |
| pH | 4,5 - 5,5 |

Der Extrakt ist unkonserviert oder auch konserviert herstell- und lagerbar. Seine Herstellung wird in der Internationalen Patentanmeldung WO 02/102347 beschrieben.

Eine mögliche Konservierung ist beispielsweise mit Butylenglycol 0,36% und Parabenen 0,14% und ist in Ethanol bis zu einem Verhältnis von 40/60 Ethanol/Wasser (v/v) löslich. Bei der Herstellung werden die Anis-Früchte in Wasser solubilisiert und enzymatisch hydrolysiert. Das Verhältnis von Rohmaterial zu Extrakt beträgt 1 : 2. Lösliche und unlösliche Phasen werden getrennt und anschließend filtriert bzw. danach steril filtriert.

Weißer Teeextrakt wird in der Regel aus einem lediglich zu 2% anfermentierten Tee gewonnen. Diese Fermentation vollzieht sich während des Welkprozesses auf natürlichem Wege. Beim weißen Tee werden die zarten Blütenknospen dieser Spezies getrocknet. Bei der Trocknung werden sie weißlich - daher auch der Name dieses Tees. Er wird oft als spezielle Sorte des Grünen Tees bezeichnet.

Weißer Tee wird mittlerweile in fast allen Teeregionen hergestellt; außer China z.B. auch in Assam, Darjeeling, Nilgiri, Sri Lanka, Malawi, Kenia. Der Charakter ist regional sehr verschieden und die Extrakte weisen daher auch eine wechselnde Zusammensetzung auf.

Weißer Tee gehört zur Spezies mit der INCI Camellia Sinensis. Wesentliche Bestandteile des Weißen Teeextraktes sind Xanthine und Catechine, speziell Gallocatechin, Epigallocatechin,Epicatechin, Epigallocatechingallat, Gallocatechingallat und Epicatechingallat.

Weißer Teeextrakt wird von Cognis unter Extrapone® White Tea angeboten.

Die Liste der genannten Extraktionsverfahren soll selbstverständlich nicht limitierend sein. Die Erfindungsgemäß Kombinierten Anisextrakte und Weißen Teeextrakte sind auf zahlreichen, an sich bekannten Wegen erhältlich. Wesentlich dabei ist, dass die Kombinierten Anisextrakte und Weißen Teeextrakte die erfindungsgemäßen Eigenschaften zeigen.

Typischerweise enthält erfindungsgemäßer Anisextrakt und Weißen Teeextrakt einige oder alle der folgenden Inhaltsstoffe; Flavonoide (Glucoside von Quercitin, Kämpferol), Sterole, Tannine, Aminosäuren, ätherische Öle und Catechine.

Die erfindungsgemäßen kosmetischen Formulierungen können wie üblich zusammengesetzt sein und zur Behandlung der Haut und/oder der Haare im Sinne der pflegenden Kosmetik dienen. Sie können aber auch in Schminkprodukten in der dekorativen Kosmetik eingesetzt werden.

Besonders bevorzugt stellen die erfindungsgemäßen Zubereitungen O/W-Emulsionen dar.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, jeweils bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**W/O-Emulsionen**

| | 1 | 2 | 3 | 4 | 5 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2.5 |
| hydrierte Kokosglyceride | 2,0 | 1,0 | 2.5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Iminodibernsteinsäure | 0,5 | --- | --- | -- | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Weißer Teeextrakt | 5 | 15 | 8 | 10 | 3 |
| Parfum | q,s, | q,s, | q,s, | q,s, | q,s, |
| Ethanol | 2,0 | --- | 5.0 | --- | --- |
| Capryl-/Caprinsäutetriglycerid | 2,0 | 2,5 | 3,0 | 5,0 | 0,5 |
| Methylparaben | 0,4 | 0,15 | 0,05 | 0,3 | 0,4 |
| Propylparaben | 0,3 | 0,4 | 0,25 | 0,15 | --- |
| Anisextrakt | 5 | 15 | 8 | 10 | 3 |
| Wasser | ad 100 | ad 100 | ad 100 | ad 100 | ad 100 |

**W/O-Emusion**

| | 6 | 7 | 8 | 9 | 10 |
|---|---|---|---|---|---|
| Cetyldimethiconecopolyol | 1,0 | --- | --- | --- | 5,0 |
| PEG-30 Dipolyhydroxystearat | --- | 0,5 | 5,0 | --- | --- |
| Lanolin Alcohol | 1,0 | 1,5 | 1,75 | 3.0 | --- |
| Cylomethicon + Dimethiconcopolyol | 10,0 | 12,5 | --- | --- | --- |
| Cyclomethicon | 12,5 | 15 | 8,0 | --- | 17,5 |
| Dimethicon | 5,0 | 13,0 | 5,0 | --- | 15,0 |
| Medizinisches Weißöl | 0,5 | 0,75 | 1,0 | 18,0 | 0,25 |
| Octyldodecanol | 0,5 | 1.0 | 0,75 | 3,0 | 0,25 |
| Panthenol | 0,5 | 1,0 | 0,75 | 0,25 | 0,1 |
| Magnesiumchlorid | 2,0 | 0,6 | 2,5 | 0,7 | 1,0 |
| Glycerin | 12,5 | 5,0 | 10,0 | 5,0 | 7,5 |
| Weißer Teeextrakt | 6 | 10 | 12 | 1,5 | 5,0 |
| Parfum | q,s, | q,s, | q,s, | q,s, | q,s, |
| Methylparaben | 0,4 | 0,1 | 0,05 | 0,3 | 0,4 |
| Butylenglykol | --- | 5,0 | --- | -- | 7,5 |
| Propylparaben | 0,3 | 0,4 | 0,25 | 0,15 | --- |
| Cetyldimethicon | 0,5 | --- | 0,7 | --- | --- |
| Anisextrakt | 6 | 10 | 12 | 1,5 | 5,0 |
| Wasser | ad 100 | ad 100 | ad 100 | ad 100 | ad 100 |

**O/W-Emulsionen**

| | 11 | 12 | 13 | 14 | 15 |
|---|---|---|---|---|---|
| Glycerylstearatcitrat | 5 | --- | --- | --- | --- |
| Glycerylstearat | --- | 5 | --- | --- | --- |
| PEG-40-Stearat | --- | 2 | --- | --- | --- |
| Polyethylenglycol(21)stearylether | --- | --- | 2 | --- | --- |
| Polyethylenglycol(2)stearylether --- | --- | --- | 1 | --- | --- |
| Cetearylglucosid | --- | --- | --- | 2 | --- |
| Stearinsäure | --- | --- | --- | --- | 5 |
| Behenylalkohol | --- | --- | --- | --- | 2 |
| Stearylalkohol | 2 | 1 | --- | 5 | -- |
| Cetylstearylalkohol | --- | --- | 2 | --- | 1 |
| Hydrierte Cocosfettglyceride | 2 | --- | --- | 3 | 1 |
| Butylenglycol Dicaprylat/Dicaprat | 1 | --- | 8 | --- | 2 |
| Caprylic/Capric Triglyceride | --- | 4 | 2 | --- | --- |
| Ethylhexylkokosfettsäureester | 3 | 6 | --- | --- | 2 |
| Octyldodecanol | --- | --- | 1 | 9 | --- |
| Mineralöl | 9 | 1 | 1 | 1 | 3 |
| Vaseline | 4 | 2 | 5 | 0,5 | 0,75 |
| Glycerin | 7,5 | 15 | 65 | 25 | --- |
| Weißer Teeextrakt | 1 | 3 | 8,5 | 0,5 | 1,5 |
| Anisextrakt | 1 | 3 | 8,5 | 0,5 | 2 |
| Octamethyltetrasitoxan | --- | 1 | 2 | 5 | --- |
| Dimethylpolysiloxan | 0,5 | 0,75 | 1,25 | --- | 1 |
| Dicarprylylcarbonat | 6 | 2 | 10 | 0,5 | 4 |
| Methylparaben | 0,3 | --- | 0,1 | --- | 0,05 |
| Parfüm | q.s. | q.s. | q.s. | q.s. | q.s. |
| Wasser | Ad 100 | Ad 100 | Ad 100 | Ad 100 | Ad 100 |

**Hydrodispersionssgel**

| | 16 | 17 | 18 | 19 | 20 |
|---|---|---|---|---|---|
| Silikonöl, cyclisch | 8 | 10 | 5 | 3 | --- |
| Silikonöl, linear | --- | --- | --- | --- | 3 |
| Dimethiconol | 1 | 2 | 3 | --- | 3 |
| Ethanol | 10 | 15 | 7,5 | 5 | 3 |
| Weißer Teeextrakt | 15 | 5 | 15 | 20 | 3,0 |
| Anisextrakt | 15 | 5 | 15 | 20 | 3,0 |
| Natriumpolyacrylat | 0,2 | 0,3 | 0,3 | 0,4 | 0,10 |
| Methylpropandiol | 2 | 3 | 4 | 5 | --- |
| Glycerin | 9 | 15 | 5 | 7,5 | 25 |
| Carbomer | 0,2 | 0,3 | 0,2 | 0,4 | 0,15 |
| Acrylates/C10-30Alkyl Acrylate Crosspolymer | 0,2 | 0,15 | 0,3 | 0,4 | 0,10 |
| Carrageenan (Chondrus Crispus) | 0,2 | --- | -- | 1,0 | 2 |
| Hamamelis Extrakt | 0,1 | 0,2 | 0,3 | 0,4 | --- |
| Parfum | q.s. | q.s. | q.s. | q.s. | q.s. |
| Farbstoff | q.s. | q.s. | q.s. | q.s. | q.s. |
| Wasser | ad 100 | ad 100 | ad 100 | ad 100 | ad 100 |
| Aloe Vera Extrakt | 0,1 | 0,2 | 0,5 | 0,2 | 1,0 |

**Ethanolisches Spray**

| | 21 | 22 | 23 | 24 | 25 |
|---|---|---|---|---|---|
| Glycerin | 9 | 15 | 20 | 5 | 7,5 |
| Ethanol | 42,5 | 45 | 40 | 35 | 25 |
| Weißer Teeextrakt | 1,5 | 10 | 5 | 2,0 | 25 |
| Anisextrakt | 1,5 | 10 | 5 | 2,0 | 25 |
| Wasser | ad 100 | ad 100 | ad 100 | ad 100 | ad 100 |
| PPG-1-PEG-9-Laurylglycolether | 0,4 | 0,2 | 0,6 | 1,0 | 5 |
| Parfüm | q.s. | q.s. | q.s. | q.s. | q.s. |
| Hamamelis Extrakt | 0,1 | 0,2 | 0,35 | 0,4 | --- |
| Aloe Vera Extrakt | 0,025 | 0,2 | 0,5 | 0,02 | 1,0 |

**Hautpflegeöle**

| | 26 | 27 | 28 | 29 | 30 |
|---|---|---|---|---|---|
| Jojobaöl | 5,0 | 7,5 | 10,0 | 25 | 25 |
| Hydriertes PEG-40 Rizinusöl | 2,5 | 7,5 | 10,0 | 1,25 | 5 |
| Macadamianußöl | 5,0 | 10,0 | 20,0 | 25,0 | 5,0 |
| Anisextrakt | 1,0 | 2,5 | 2,0 | 0,1 | 0,25 |
| Cyclomethicon | --- | --- | --- | --- | 1,5 |
| Dimethicon | --- | 0,25 | --- | --- | --- |
| Weißer Teeextrakt | 1,0 | 2,5 | 0,75 | 0,25 | 1,5 |
| medizinisches Weißöl | 30,0 | --- | --- | --- | 5,0 |
| Caprylic/Capric Triglycerid | 5,0 | --- | --- | --- | --- |
| Rizinusöl | 5,0 | --- | --- | --- | 5,0 |
| Octyldodecanol | --- | 1,0 | --- | 10,5 | 0,25 |
| Parfum | q,s, | q,s, | 0,1 | q,s, | --- |
| Isopropylpalmitat | --- | --- | --- | --- | --- |
| Ethylhexylstearat | 10,0 | --- | --- | --- | --- |
| Tocopherolacetat | 0,1 | --- | --- | 0,15 | --- |
| Tocopherol | 0,05 | 0,15 | 0,05 | 0,05 | 0,05 |
| Sonnenblumenöl | ad 100 | ad 100 | ad 100 | ad 100 | ad 100 |

**O/WEmulsionen, lelcht**

| | 31 | 32 | 33 | 34 | 35 |
|---|---|---|---|---|---|
| Weißer Teeextrakt | 15 | 10 | 5 | 20 | 30 |
| Glycerylstearat | 1 | 1 | 2 | 5 | 8 |
| Cetearylalkohol + Natriumcetearylsulfat | 3 | 4 | 5 | 2 | 8 |
| Anisextrakt | 15 | 10 | 5 | 20 | 30 |
| Xanthan Gummi | 0,5 | 0,75 | 0,35 | 0,4 | 1,0 |
| Parfum | q.s. | q.s. | q.s. | q.s. | q.s. |
| Hamamelis Extrakt | 0,1 | 0,2 | 0,35 | 0,4 | --- |
| Aloe Vera Extrakt | 0,025 | 0,2 | 0,5 | 0,02 | 1,0 |
| Dicaprylylcarbonat | 5 | 7,5 | 3 | 10 | 2,5 |
| Caprylic/Capric Trilycerid | 5 | 7,5 | 3 | 2,1 | 1,8 |
| Silikonöl, linear | 3 | 2 | --- | 0,75 | 5 |
| Caprylylglykol | 0,5 | --- | 0,3 | --- | 0,1 |
| Wasser | ad 100 | ad 100 | ad 100 | ad 100 | ad 100 |
| Ethanol | 2,5 | 3,5 | 5 | 1,5 | --- |

**Gele**

| | 36 | 37 | 38 | 39 | 40 |
|---|---|---|---|---|---|
| Polyacrylsäure, Na-Salz | 1,0 | 0,2 | 0,25 | 0,1 | 1,5 |
| PEG-40-Stearat | 0,5 | --- | --- | 0,75 | --- |
| Carrageenan | 1,25 | 0,2 | 0,2 | 0,1 | 2,5 |
| Natriumpolyacrylat | 0,2 | 0,2 | 0,2 | 0,5 | 1,5 |
| Vinylpyrrolidon/Vinylacetat-Copolymeren | 1,0 | --- | --- | 0,2 | --- |
| Acrylates/C10-C30 Alkyl Acrylate Crosspolymer | 1,5 | 0,2 | 0,25 | 1,5 | 0,75 |
| Wasserlösliches Blau (Cl 42090) | 0,001 | 0,4 | 0,0001 | 0,003 | --- |
| Wasserlösliches Gelb (Cl 10316) | 0,01 | 0,004 | 0,0001 | --- | 0,002 |
| Cyclomethicon | 1 | 8,5 | 8,0 | 0,5 | 3 |
| Dimethiconol | 0,5 | 0,5 | 1,0 | 1,25 | 1,0 |
| Cetylstearylalkohol | --- | --- | --- | 1 | --- |
| hydriertes Polyisobuten | 0,5 | --- | --- | --- | 0,25 |
| Weißer Teeextrakt | 0,5 | 0,1 | 0,75 | 0,5 | 0,25 |
| Anisextrakt | 1,0 | 2,0 | 0,5 | | |
| Panthenol | 0,5 | --- | --- | 0,25 | 0,1 |
| Glycerin | 3,0 | 8,6 | 10,0 | 17,2 | 5,0 |
| Parfum | q,s, | q,s, | q,s, | q,s, | q,s, |
| Methylproparidiol | 4,5 | 2,0 | 2,0 | 1,5 | --- |
| Phenoyethanol | 0,1 | 1,0 | 0,3 | --- | 0,5 |
| Propylparaben | 0,3 | --- | --- | 0,15 | --- |
| Ethanol | --- | 10,0 | 10,0 | --- | 3,5 |
| Wasser | ad 100 | ad 100 | ad 100 | ad 100 | ad 100 |

## Patentansprüche

1. Kosmetische Zubereitungen, enthaltend Wirkstoffkombinationen aus einer wirksamen Menge an Anisfruchtextrakt und Weißem Teeextrakt.

2. Zubereitung nach Anspruch 1, **dadurch gekennzeichnet, daß** die Konzentration an Anisfruchtextrakt von 0,001 bis 30 Gew.-% gewählt wird.

3. Zubereitung nach einem der vorhergehenden Anspruche, **dadurch gekennzeichnet, daß** die Konzentration an Anisfruchtextrakt von 0,5 bis 10 Gew.-% gewählt wird.

4. Zubereitung nach Anspruch 1, **dadurch gekennzeichnet, daß** die Konzentration an weißem Teeextrakt von 0,001 bis 30 Gew.-% gewählt wird.

5. Zubereitung nach einem der vorhergehenden Ansprüche, **dadurch gekennzeichnet, daß** die Konzentration an Weißem Teeextrakt von 0,5 bis 10 Gew.-% gewählt wird.

6. Zubereitungen nach einem der vorgehenden Ansprüche, **dadurch gekennzeichnet, daß** Gewichtsverhältnisse von Anisfruchtextrakt und weißem Teeextrakt aus den Bereichen 1 : 10 bis 10 : 1, bevorzugt von 1 :5 bis 5 :1, besonders bevorzugt von 1 : 2 bis 2 : 1, gewählt werden.

7. Verwendung von kosmetischen Zubereitungen nach einem der vorhergehenden Ansprüche zur Steigerung der Hautfeuchtigkeit bzw. zur Befeuchtung der Haut.

8. Verwendung von kosmetischen Zubereitungen nach einem der vorhergehenden Ansprüche zur Verbesserung der Hautoberflächenstruktur, insbesondere zur Glättung der Haut und zur Verringerung von Hautdellen.

9. Verwendung von kosmetischen Zubereitungen nach einem der vorhergehenden Ansprüche zur Verminderung von Cellulite.

10. Verwendung von kosmetischen Zubereitungen nach einem der vorhergehenden Ansprüche zur Pflege sensibler Haut.

## Claims

1. Cosmetic preparations comprising active agent combinations made from an effective amount of anise fruit extract and white tea extract.

2. Preparation according to Claim 1, **characterized in that** the concentration of anise fruit extract is chosen from 0.001 to 30% by weight.

3. Preparation according to one of the preceding claims, **characterized in that** the concentration of anise fruit extract is chosen from 0.5 to 10% by weight.

4. Preparation according to Claim 1, **characterized in that** the concentration of white tea extract is chosen from 0.001 to 30% by weight.

5. Preparation according to one of the preceding claims, **characterized in that** the concentration of white tea extract is chosen from 0.5 to 10% by weight.

6. Preparations according to one of the preceding claims, **characterized in that** weight ratios of anise fruit extract and white tea extract are chosen from the ranges 1:10 to 10:1, preferably from 1:5 to 5:1, particularly preferably from 1:2 to 2:1.

7. Use of cosmetic preparations according to one of the preceding claims for increasing the skin moisture and for moisturizing the skin.

8. Use of cosmetic preparations according to one of the preceding claims for improving the structure of the skin surface, in particular for smoothing the skin and for reducing skin dimples.

9. Use of cosmetic preparations according to one of the preceding claims for reducing cellulite.

10. Use of cosmetic preparations according to one of the preceding claims for the care of sensitive skin.

## Revendications

1. Préparations cosmétique, contenant des associations de substances actives à base d'une quantité efficace d'extrait de fruit d'anis et d'extrait de thé blanc.

2. Préparation selon la revendication 1, **caractérisée en ce que** la concentration de l'extrait de fruit d'anis est choisie de 0,001 à 30 % en poids.

3. Préparation selon l'une quelconque des revendications précédentes, **caractérisée en ce que** la concentration de l'extrait de fruit d'anis est choisie de 0,5 à 10 % en poids.

4. Préparation selon la revendication 1, **caractérisée en ce que** la concentration de l'extrait de thé blanc est choisie de 0,001 à 30 % en poids.

5. Préparation selon l'une quelconque des revendications précédentes, **caractérisée en ce que** la concentration de l'extrait de thé blanc est choisie de 0,5 à 10 % en poids.

6. Préparation selon l'une quelconque des revendications précédentes, **caractérisée en ce que** les rapports pondéraux de l'extrait de fruit d'anis et d'extrait de thé blanc sont choisis dans les plages allant de 1 : 10 à 10 : 1, de préférence de 1 : 5 à 5 : 1, de façon particulièrement préférée de 1 : 2 à 2 : 1.

7. Utilisation de préparations cosmétiques selon l'une quelconque des revendications précédentes, pour l'augmentation de l'humidité de la peau ou pour l'hydratation de la peau.

8. Utilisation de préparations cosmétiques selon l'une quelconque des revendications précédentes, pour l'amélioration de la texture superficielle de la peau, en particulier pour le lissage de la peau et pour la diminution de capitons cutanés.

9. Utilisation de préparations cosmétiques selon l'une quelconque des revendications précédentes, pour la diminution de la cellulite.

10. Utilisation de préparations cosmétiques selon l'une quelconque des revendications précédentes, pour le soin de la peau sensible.
